# EUROPÄISCHE PATENTSCHRIFT

(11) **EP 2 866 748 B1**
(45) Veröffentlichungstag und Bekanntmachung des Hinweises auf die Patenterteilung: **07.12.2016**
(21) Anmeldenummer: 13736812.2
(22) Anmeldetag: 28.06.2013
(51) Int. Cl.: A61F 5/02

(54) **VARIABLE RÜCKENSCHALE MIT ZWEI SEITENSCHALENTEILEN**
VARIABLE BACK SHELL HAVING TWO SIDE SHELL PARTS
COQUE DORSALE MODIFIABLE COMPORTANT DEUX PARTIES DE COQUE LATÉRALES

(30) Priorität: 29.06.2012 DE 102012013175
(43) Veröffentlichungstag der Anmeldung: 06.05.2015
(73) Patentinhaber: Bauerfeind AG, 07937 Zeulenroda-Triebes (DE)
(72) Erfinder: RÖBELT, Gerhard, 07950 Zeulenroda-Triebes (DE); STIER, Gerald, 07957 Langenwetzendorf (DE); BAETZ, Ronny, 07937 Vogtländisches Oberland OT, Pöllwitz (DE)
(74) Vertreter: Wohlfahrt, Jan Günther
(86) Internationale Anmeldenummer: PCT/EP2013/063606
(87) Internationale Veröffentlichungsnummer: WO 2014/001493

(56) Entgegenhaltungen:
- FR-A1- 2 968 540
- US-A- 4 735 196
- US-A1- 2004 220 503

## Beschreibung

Die Erfindung betrifft orthopädische Rückenschalen, die als Rückenorthesen oder als Halterung für andere Orthesen, beispielsweise Schulterorthesen, verwendet werden können. Die Erfindung betrifft auch eine Orthese, welche eine erfindungsgemäße Rückenschale enthält. Offenbart ist auch die orthopädische Verwendung der Rückenschalen.

Orthesen sind therapeutische Hilfsmittel zur Stabilisierung oder Unterstützung der Bewegungsfunktion von Körperteilen. Bei der Versorgung mit funktionellen Orthesen steht die Wiederherstellung und Erhaltung der Mobilität des Bewegungsapparates im Vordergrund. Orthesen können funktionssichernd, mobilisierend, stützend, bewegungslimitierend und/oder korrigierend wirken. Der Einsatz der Orthesen kann direkt posttraumatisch oder postoperativ oder konservativ erfolgen, d. h. zum Wiederaufbau, zur Sicherung oder zum Schutz der Gelenkfunktionen, z. B. bei degenerativen Erkrankungen. Um die je nach Anwendung erforderliche Stabilität zu erreichen, sind in der Orthese eines oder mehrere Stabilisierungselemente vorgesehen. Diese sind in der Regel im Wesentlichen stabförmig oder schalenförmig ausgebildet. Die als Schalen ausgebildeten Stabilisierungselemente umgreifen das zu stabilisierende Körperteil oder Körpergelenk, beispielsweise den Torso im Bereich der Lendenwirbelsäule, um mehr als die Hälfte dessen Umfangs.

Rückenorthesen sind beispielsweise aus den Druckschriften DE 19855923 A1 und DE 20 2005 011 650 U1 bekannt. Eine einteilige orthopädische Rückenschalen ist aus der DE 10 2009 056 710 A1 bekannt. Die WO 97/03627 A1 beschreibt eine Rückenschale, die über Gurte mit einer Bauchschale verbunden ist.

Die US 2004/0220503 offenbart eine mehrteilige Rückenschale, wobei die einzelnen Teile gegeneinander verschiebbar sind.

Das der Erfindung zugrunde liegende technische Problem besteht primär darin, orthopädische Rückenschalen bereitzustellen, die einfach und genau an den Körperumfang und Körperbau, insbesondere im Lendenwirbel- und Beckenbereich, des Trägers, beispielsweise des Patienten, anpassbar sind, wobei gleichzeitig ein guter Tragekomfort gegeben sein soll. Weiter soll eine orthopädische Rückenschale bereitgestellt werden, die bei Schaffung einer anatomisch korrekten Stabilität gleichzeitig ein weites Spektrum an Körpergrößen abdecken kann.

Das technische Problem wird vollständig gelöst durch die Bereitstellung einer orthopädische Rückenschale, wobei die Rückenschale mindestens ein erstes flexibles Seitenschalenteil und ein zweites flexibles Seitenschalenteil aufweist, wobei das erste Seitenschalenteil und das zweite Seitenschalenteil über mindestens zwei Verbindungselemente miteinander verbunden sind und wobei das erste Seitenschalenteil und das zweite Seitenschalenteil über die mindestens zwei Verbindungselemente variabel aneinander fixiert werden können, gemäß den weiteren Merkmalen von Anspruch 1.

In einer ebenfalls offenbarten Ausführungsform kann die Rückenschale zusätzlich mindesten ein Mittelschalenteil aufweisen.

Offenbart ist auch eine orthopädische Rückenschale, wobei die Rückenschale mindestens ein erstes flexibles Seitenschalenteil, ein zweites flexibles Seitenschalenteil und ein Mittelschalenteil aufweist, wobei das erste Seitenschalenteil und das zweite Seitenschalenteil über jeweils mindestens ein Verbindungselement mit dem Mittelschalenteil verbunden sind und wobei das erste Seitenschalenteil und das zweite Seitenschalenteil über das mindestens eine Verbindungselement variabel an das Mittelschalenteil fixiert werden können.

In einer ebenfalls offenbarten Ausführungsform können das erste Seitenschalenteil und das zweite Seitenschalenteil über überlappende Flächenbereiche mit dem Mittelschalenteil verbunden sein.

Offenbart ist also eine orthopädische Rückenschale, die mehrere Schalenteile, beispielsweise zwei oder drei Schalenteile, aufweist, wobei aneinanderliegende oder sich überlappende Schalenteile variabel über jeweils mindestens ein Verbindungselement miteinander verbunden sind und über das mindestens eine Verbindungselement zueinander fixiert werden können.

Die Schalen können also variabel zueinander positioniert werden, so dass die Rückenschale einfach und genau an den Körperumfang und Körperbau, insbesondere im Lendenwirbel- und Beckenbereich, des Trägers angepasst werden kann und dann in der gewünschten Position zueinander fixiert werden, so dass die Rückenschale an den Körperumfang und Körperbau angepasst bleibt.

Natürlich kann die Fixierung bei Bedarf wieder aufgehoben werden, beispielsweise wenn sich der Köperumfang ändert.

Bei dem ersten Seitenschalenteil kann es sich um das linke oder um das rechte Seitenschalenteil handeln. Bei dem zweiten Seitenschalenteil handelt es sich dann um das demgemäß andere Seitenschalenteil, also um das rechte oder das linke.

Das ebenfalls offenbarte Mittelschalenteil ist bevorzugt mittig am Rücken des Patienten positioniert.

In einer bevorzugten Ausführungsform sind das erste Seitenschalenteil und das zweite Seitenschalenteil gleich dimensioniert, so dass aus den zwei Seitenschalenteilen oder den zwei Seitenschalenteilen und dem mindestens einen Mittelschalenteil eine in etwa achsensymmetrische Rückenschale gebildet werden kann.

In einer bevorzugten Ausführungsform können das erste Seitenschalenteil und das zweite Seitenschalenteil jeweils mindestens einen Teil des Rückens und eine Taillenseite einer Person umfassen.

In einer bevorzugten Ausführungsform sind das erste Seitenschalenteil und das zweite Seitenschalenteil über überlappende Flächenbereiche miteinander verbunden sind.

In einer bevorzugten Ausführungsform sind die Schalenteile, insbesondere das erste Seitenschalenteil und das zweite Seitenschalenteil aus einem flexiblen Kunststoffmaterial ausgebildet. Ein geeignetes Kunststoffmaterial besteht zum Beispiel aus mindestens einem Polyamid oder enthält Polyamide. Dem Fachmann sind weitere geeignete Materialien, insbesondere Kunststoffmaterielaien bekannt, die eine geeignete Flexibilität aufweisen, beispielsweise Gießharze.

In einer bevorzugten Ausführungsform sind das erste Seitenschalenteil und das zweite Seitenschalenteil mittels Kunststoff-Spritzgießtechnik hergestellt. Im Stand der Technik sind geeignete Kunststoff-Spritzgießtechniken zur Herstellung von Teilen von Orthesen, auch von Stabilisierungselementen für Orthesen bekannt.

Auch das ebenfalls offenbarte Mittelschalenteil kann aus einem flexiblen Kunststoffmaterial ausgebildet sein. Es kann aber auch aus einem anderen Material hergestellt sein. Bevorzugt ist das Mittelschalenteil ebenfalls flexibel. Es kann aber auch starr sein, beispielsweise im Bereich der Wirbelsäule.

In einer bevorzugten Ausführungsform weist das erste Seitenschalenteil und das zweite Seitenschalenteil mindestens drei Löcher zum Fixieren des mindestens einen Verbindungssystems auf.

Die Löcher können entweder Rundlöcher oder erfindungsgemäß Langlöcher sein. Bei der Verwendung von Rundlöchern kann die Variabilität der Fixierung insbesondere durch die Anzahl der Rundlöcher erreicht werden. Bei der erfindungsgemäßen Verwendung von Langlöchern kann die Variabilität der Fixierung insbesondere durch die Länge, Krümmung und Positionierung der Langlöcher erreicht werden.

In einer offenbarten Ausführungsform weist das erste Seitenschalenteil und das zweite Seitenschalenteil eine Vielzahl von Löchern, insbesondere Rundlöchern zum Fixieren des mindestens einen Verbindungssystems auf. In einer offenbarten Ausführungsform weist das Mittelschalenteil eine Vielzahl von Löchern, insbesondere Rundlöchern zum Fixieren des mindestens einen Verbindungssystems auf.

Die Schalen mit einer Vielzahl von Löchern können beispielsweise als schalenförmige Lochplatte ausgebildet sein.

Bevorzugt ist das mindestens eine Verbindungselement als Schrauben-Mutter Verbindung, als Schnellspannvorrichtung oder als Nietenverbindung ausgebildet.

In einer bevorzugten Ausführungsform ist das mindestens eine Verbindungselement als Clipverbindung oder als schiebbare Rastverbindung ausgebildet.

Offenbart ist eine orthopädischen Rückenschale, wobei die Rückenschale mindestens ein erstes flexibles Seitenschalenteil und mindestens ein zweites flexibles Seitenschalenteil aufweist, wobei das erste Seitenschalenteil und das zweite Seitenschalenteil über mindestens zwei Verbindungselemente miteinander verbunden sind und wobei das erste Seitenschalenteil und das zweite Seitenschalenteil über die mindestens zwei Verbindungselemente variabel aneinander fixiert werden können, wobei sowohl das erste Seitenschalenteil mindestens zwei übereinander angeordnete Langlöcher als auch das zweite Seitenschalenteil mindestens zwei übereinander angeordnete Langlöcher aufweisen und das erste Seitenschalenteil und das zweite Seitenschalenteil über die mindestens zwei Verbindungselemente miteinander verbunden sind, wobei jeweils eins der zwei Verbindungselemente durch ein Langloch des ersten Seitenschalenteils und ein Langloch des zweiten Seitenschalenteils hindurchreicht, und wobei das oberste Langloch des ersten Seitenschalenteils und das oberste Langloch des zweiten Seitenschalenteils einen unterschiedlichen Winkel haben und wobei das unterste Langloch des ersten Seitenschalenteils und das unterste Langloch des zweiten Seitenschalenteils einen unterschiedlichen Winkel haben.

In einer bevorzugten Ausführungsform weisen sowohl das erste Seitenschalenteil mindestens drei übereinander angeordnete Langlöcher als auch das zweite Seitenschalenteil mindestens drei übereinander angeordnete Langlöcher auf.

In einer bevorzugten Ausführungsform haben das mittlere Langloch des ersten Seitenschalenteils und das mittlere Langloch des zweiten Seitenschalenteils einen unterschiedlichen Winkel.

Erfindungsgemäß sind das oberste Langloch und das unterste Langloch des ersten Seitenschalenteils senkrecht oder nahezu senkrecht angeordnet, wobei das oberste Langloch und das unterste Langloch des zweiten Seitenschalenteils waagerecht oder nahezu waagerecht angeordnet sind.

In einer bevorzugten Ausführungsform sind das mittlere Langloch des ersten Seitenschalenteils und das mittlere Langloch des zweiten Seitenschalenteils schräg angeordnet.

In einer bevorzugten Ausführungsform sind das mittlere Langloch des ersten Seitenschalenteils und das mittlere Langloch des zweiten Seitenschalenteils schräg zueinander angeordnet.

In einer bevorzugten Ausführungsform sind das oberste Langloch und das unterste Langloch des ersten Seitenschalenteils senkrecht oder nahezu senkrecht angeordnet wobei das oberste Langloch und das unterste Langloch des zweiten Seitenschalenteils waagerecht oder nahezu waagerecht angeordnet sind und wobei das mittlere Langloch des ersten Seitenschalenteils und das mittlere Langloch des zweiten Seitenschalenteils schräg angeordnet sind.

In einer bevorzugten Ausführungsform besteht die orthopädische Rückenschale aus dem ersten flexiblen Schalenteil und dem zweiten flexiblen Schalenteil. In einer bevorzugten Ausführungsform weist die orthopädische Rückenschale kein Mittelschalenteil auf. In einer bevorzugten Ausführungsform ist das erste Seitenschalenteil mit dem zweiten Seitenschalenteil über überlappende Flächenbereiche miteinander verbunden.

In einer bevorzugten Ausführungsform weist das erste Seitenschalenteil oder das zweite Seitenschalenteil zwei Langlöcher oder drei Langlöcher auf. In einer bevorzugten Ausführungsform weist das erste Seitenschalenteil und das zweite Seitenschalenteil zwei Langlöcher oder drei Langlöcher auf.

Erfindungsgemäß weist das erste Seitenschalenteil und/oder das zweite Seitenschalenteil mindestens zwei Langlöcher auf, wobei das erste Seitenschalenteil und das zweite Seitenschalenteil über mindestens zwei Verbindungselemente miteinander verbunden sind, wobei je ein Verbindungselement durch je ein Langloch hindurchreicht.

In einer bevorzugten Ausführungsform weist das erste Seitenschalenteil der orthopädischen Rückenschale mindestens drei Langlöcher auf.

In einer bevorzugten Ausführungsform weisen sowohl das erste Seitenschalenteil der orthopädischen Rückenschale mindestens drei Langlöcher als auch das zweite Seitenschalenteil mindestens drei Langlöcher auf.

In einer bevorzugten Ausführungsform weisen sowohl das erste Seitenschalenteil der orthopädischen Rückenschale mindestens drei Langlöcher als auch das zweite Seitenschalenteil mindestens drei Langlöcher auf und das erste Seitenschalenteil und das zweite Seitenschalenteil sind über mindestens drei Verbindungselemente miteinander verbunden, wobei jeweils eins der drei Verbindungselemente durch ein Langloch des ersten Seitenschalenteils und ein Langloch des zweiten Seitenschalenteils hindurchreicht.

Die vorliegende Erfindung löst das technische Problem insbesondere auch durch die Bereitstellung einer erfindungsgemäßen orthopädischen Rückenschale, wobei sowohl das erste Seitenschalenteil mindestens drei übereinander angeordnete Langlöcher als auch das zweite Seitenschalenteil mindestens drei übereinander angeordnete Langlöcher aufweisen und das erste Seitenschalenteil und das zweite Seitenschalenteil über mindestens drei Verbindungselemente miteinander verbunden sind, wobei jeweils eins der drei Verbindungselemente durch ein Langloch des ersten Seitenschalenteils und ein Langloch des zweiten Seitenschalenteils hindurchreicht.

Bevorzugt haben das oberste Langloch des ersten Seitenschalenteils und das oberste Langloch des zweiten Seitenschalenteils einen unterschiedlichen Winkel, wobei das mittlere Langloch des ersten Seitenschalenteils und das mittlere Langloch des zweiten Seitenschalenteils einen unterschiedlichen Winkel haben und wobei das unterste Langloch des ersten Seitenschalenteils und das unterste Langloch des zweiten Seitenschalenteils einen unterschiedlichen Winkel haben.

Erfindungsgemäß sind die mindestens zwei Langlöcher des ersten Seitenschalenteils und die mindestens zwei Langlöcher des zweiten Seitenschalenteils jeweils übereinander angeordnet.

In einer bevorzugten Ausführungsform sind die drei Langlöcher des ersten Seitenschalenteils und die drei Langlöcher des zweiten Seitenschalenteils jeweils übereinander angeordnet.

Erfindungsgemäß haben das oberste Langloch des ersten Seitenschalenteils und das oberste Langloch des zweiten Seitenschalenteils einen unterschiedlichen Winkel, wobei das unterste Langloch des ersten Seitenschalenteils und das unterste Langloch des zweiten Seitenschalenteils einen unterschiedlichen Winkel haben.

In einer bevorzugten Ausführungsform haben das oberste Langloch des ersten Seitenschalenteils und das oberste Langloch des zweiten Seitenschalenteils einen unterschiedlichen Winkel, wobei das mittlere Langloch des ersten Seitenschalenteils und das mittlere Langloch des zweiten Seitenschalenteils einen unterschiedlichen Winkel haben und wobei das unterste Langloch des ersten Seitenschalenteils und das unterste Langloch des zweiten Seitenschalenteils einen unterschiedlichen Winkel haben.

Erfindungsgemäß sind das oberste Langloch und das unterste Langloch des ersten Seitenschalenteils senkrecht oder nahezu senkrecht angeordnet, wobei das oberste Langloch und das unterste Langloch des zweiten Seitenschalenteils waagerecht oder nahezu waagerecht angeordnet sind.

In einer offenbarten Ausführungsform weist das erste Seitenschalenteil und das zweite Seitenschalenteil mindestens jeweils zwei Langlöcher auf, wobei das erste Seitenschalenteil und das zweite Seitenschalenteil überlappen und über mindestens zwei Verbindungselemente miteinander verbunden sind, wobei je ein Verbindungselement durch je ein Langloch des ersten Seitenschalenteils und ein Langloch des zweiten Seitenschalenteils hindurchreicht. Erfindungsgemäß sind die mindestens zwei Langlöcher des ersten Seitenschalenteils und die mindestens zwei Langlöcher des zweiten Seitenschalenteils jeweils übereinander angeordnet. Erfindungsgemäß ist das oberste Langloch des ersten Seitenschalenteils senkrecht oder nahezu senkrecht angeordnet und das oberste Langloch des zweiten Seitenschalenteils waagerecht oder nahezu waagerecht angeordnet. Erfindungsgemäß ist das unterste Langloch des ersten Seitenschalenteils senkrecht oder nahezu senkrecht angeordnet und das unterste Langloch des zweiten Seitenschalenteils waagerecht oder nahezu waagerecht angeordnet. Alternativ kann auch das unterste Langloch des ersten Seitenschalenteils waagerecht oder nahezu waagerecht angeordnet sein und das unterste Langloch des zweiten Seitenschalenteils senkrecht oder nahezu senkrecht angeordnet sein.

In einer bevorzugten Ausführungsform sind das oberste Langloch und das unterste Langloch des ersten Seitenschalenteils senkrecht oder nahezu senkrecht angeordnet, wobei das oberste Langloch und das unterste Langloch des zweiten Seitenschalenteils waagerecht oder nahezu waagerecht angeordnet sind und wobei das mittlere Langloch des ersten Seitenschalenteils und das mittlere Langloch des zweiten Seitenschalenteils schräg angeordnet sind.

Offenbart ist auch eine orthopädische Rückenschale, wobei die Rückenschale ein erstes Seitenschalenteil, ein zweites Seitenschalenteil und ein Mittelschalenteil aufweist, wobei das erste Seitenschalenteil, das das zweite Seitenschalenteil und/oder das Mittelschalenteil mindestens ein Langloch aufweist und wobei das erste Seitenschalenteil und das zweite Seitenschalenteil über mindestens ein Verbindungselement mit dem Mittelschalenteil verbunden sind, wobei das Verbindungselement durch das mindestens eine Langloch hindurchreicht.

In einer offenbarten Ausführungsform sind das oberste Langloch und das unterste Langloch des ersten Seitenschalenteils und des zweiten Seitenschalenteils senkrecht oder nahezu senkrecht angeordnet sind und das oberste Langloch und das unterste Langloch des Mittelschalenteils waagerecht oder nahezu waagerecht angeordnet sind und das mittlere Langloch des ersten Seitenschalenteils, das mittlere Langloch des zweiten Seitenschalenteils schräg angeordnet sind.

In einer offenbarten Ausführungsform weist das erste und das zweite Seitenschalenteil jeweils mindestens drei Langlöcher auf und das Mittelschalenteil mindestens drei Langlöcher oder mindestens sechs Langlöcher auf.

In einer offenbarten Ausführungsform weisen sowohl das erste Seitenschalenteil der orthopädischen Rückenschale mindestens drei Langlöcher als auch das zweite Seitenschalenteil mindestens drei Langlöcher auf und das erste Seitenschalenteil und das zweite Seitenschalenteil sind über jeweils mindestens drei Verbindungselemente mit dem Mittelschalenteil verbunden, wobei jeweils eins der drei Verbindungselemente durch ein Langloch des ersten Seitenschalenteils beziehungsweise des zweiten Seitenschalenteils hindurchreicht und durch ein Loch, insbesondere Langloch des Mittelschalenteils hindurchreicht.

Offenbart ist auch eine orthopädische Rückenschale, wobei die Rückenschale mindestens ein erstes flexibles Seitenschalenteil, mindestens ein zweites flexibles Seitenschalenteil und mindestens ein Mittelschalenteil aufweist, wobei das erste Seitenschalenteil und das zweite Seitenschalenteil über jeweils mindestens zwei Verbindungselemente mit dem Mittelschalenteil verbunden sind und wobei das erste Seitenschalenteil und das zweite Seitenschalenteil über die jeweils mindestens zwei Verbindungselemente variabel an das Mittelschalenteil fixiert werden können, wobei sowohl das erste Seitenschalenteil mindestens zwei übereinander angeordnete Langlöcher als auch das zweite Seitenschalenteil mindestens zwei übereinander angeordnete Langlöcher aufweisen und wobei das Mittelschalenteil vier Langlöcher aufweist, von denen jeweils zwei übereinander angeordnet und jeweils zwei nebeneinander angeordnet sind, wobei jeweils eins der jeweils mindestens zwei Verbindungselemente durch ein Langloch des ersten Seitenschalenteils oder ein Langloch des zweiten Seitenschalenteils hindurchreicht und wobei jeweils eins der jeweils mindestens zwei Verbindungselemente durch ein Langloch des Mittelschalenteils hindurchreicht und wobei das oberste Langloch des ersten Seitenschalenteils und das oberste Langloch des zweiten Seitenschalenteils jeweils einen unterschiedlichen Winkel haben als das entsprechende mit einem Verbindungselement mit diesem verbundene Langloch des Mittelschalenteils und wobei das unterste Langloch des ersten Seitenschalenteils und das unterste Langloch des zweiten Seitenschalenteils jeweils einen unterschiedlichen Winkel haben als das entsprechende mit einem Verbindungselement mit diesem verbundene Langloch des Mittelschalenteils.

Offenbart ist auch eine orthopädische Rückenschale, wobei die Rückenschale mindestens ein erstes flexibles Seitenschalenteil, mindestens ein zweites flexibles Seitenschalenteil und mindestens ein Mittelschalenteil aufweist, wobei das erste Seitenschalenteil und das zweite Seitenschalenteil über jeweils mindestens drei Verbindungselemente mit dem Mittelschalenteil verbunden sind und wobei das erste Seitenschalenteil und das zweite Seitenschalenteil über die jeweils mindestens drei Verbindungselemente variabel an das Mittelschalenteil fixiert werden können, wobei sowohl das erste Seitenschalenteil mindestens drei übereinander angeordnete Langlöcher als auch das zweite Seitenschalenteil mindestens drei übereinander angeordnete Langlöcher aufweisen und wobei das Mittelschalenteil sechs Langlöcher aufweist, von denen jeweils drei übereinander angeordnet und jeweils zwei nebeneinander angeordnet sind, wobei jeweils eins der jeweils mindestens drei Verbindungselemente durch ein Langloch des ersten Seitenschalenteils oder ein Langloch des zweiten Seitenschalenteils hindurchreicht und wobei jeweils eins der jeweils mindestens drei Verbindungselemente durch ein Langloch des Mittelschalenteils hindurchreicht und wobei das oberste Langloch des ersten Seitenschalenteils und das oberste Langloch des zweiten Seitenschalenteils jeweils einen unterschiedlichen Winkel haben als das entsprechende mit einem Verbindungselement mit diesem verbundene Langloch des Mittelschalenteils und wobei das mittlere Langloch des ersten Seitenschalenteils und das mittlere Langloch des zweiten Seitenschalenteils jeweils einen unterschiedlichen Winkel haben als das entsprechende mit einem Verbindungselement mit diesem verbundene Langloch des Mittelschalenteilsund wobei das unterste Langloch des ersten Seitenschalenteils und das unterste Langloch des zweiten Seitenschalenteils jeweils einen unterschiedlichen Winkel haben als das entsprechende mit einem Verbindungselement mit diesem verbundene Langloch des Mittelschalenteils.

Offenbart ist auch eine orthopädische Rückenschale, wobei sowohl das erste Seitenschalenteil mindestens drei übereinander angeordnete Langlöcher als auch das zweite Seitenschalenteil mindestens drei übereinander angeordnete Langlöcher als auch das Mittelschalenteil mindestens drei übereinander angeordnete Langlöcher aufweisen und das erste Seitenschalenteil und das zweite Seitenschalenteil über jeweils mindestens drei Verbindungselemente mit dem Mittelschalenteil verbunden sind, wobei jeweils eins der jeweils mindestens drei Verbindungselemente durch ein Langloch des ersten Seitenschalenteils oder ein Langloch des zweiten Seitenschalenteils hindurchreicht und wobei jeweils eins der jeweils mindestens drei Verbindungselemente durch ein Langloch des Mittelschalenteils hindurchreicht.

Das oberste Langloch der Seitenschalenteile und das oberste Langloch des Mittelschalenteils können einen unterschiedlichen Winkel haben, wobei das mittlere Langloch der Seitenschalenteile und das mittlere Langloch des Mittelschalenteils einen unterschiedlichen Winkel haben und wobei das unterste Langloch der Seitenschalenteile und das unterste Langloch des Mittelschalenteils einen unterschiedlichen Winkel haben.

In einer bevorzugten Ausführungsform sind die drei Langlöcher des ersten Seitenschalenteils und die drei Langlöcher des zweiten Seitenschalenteils jeweils übereinander angeordnet.

In einer offenbarten Ausführungsform hat das Mittelschalenteil drei Langlöcher, die übereinander angeordnet sind. In einer alternativen Ausführungsform hat das Mittelschalenteil sechs Langlöcher, von denen jeweils drei übereinander angeordnet und jeweils zwei nebeneinander angeordnet sind.

Bei der Verwendung von Langlöchern können diese auf den Schalen unterschiedlich positioniert sein, beispielsweise senkrecht oder nahezu senkrecht oder waagerecht oder nahezu waagerecht oder schräg. Dabei können die einzelnen Langlöcher unterschiedlich positioniert sein.

In einer offenbarten Ausführungsform sind das oberste Langloch und das unterste Langloch der Seitenschalenteile senkrecht oder nahezu senkrecht angeordnet, wobei das oberste Langloch und das unterste Langloch des Mittelschalenteils waagerecht oder nahezu waagerecht angeordnet sind und wobei das mittlere Langloch der Seitenschalenteile und das mittlere Langloch des Mittelschalenteils schräg angeordnet sind.

In einer bevorzugten Ausführungsform sind waagerecht oder nahezu waagerecht angeordnete Langlöcher mindestens doppelt so lang wie senkrecht oder nahezu senkrecht angeordnete Langlöcher.

In einer bevorzugten Ausführungsform sind schräg angeordnete Langlöcher kürzer als waagerecht oder nahezu waagerecht angeordnete Langlöcher und länger als senkrecht oder nahezu senkrecht angeordnete Langlöcher.

In einer bevorzugten Ausführungsform sind die mittleren Langlöcher schräg, geradlinig und dabei zueinander spiegelbildlich zur Vertikalachse angeordnet. So befindet sich der mittlere Schnittpunkt dieser und damit auch das zugehörige Verbindungselement stets horizontal in der Mitte.

Die Position des mittleren Verbindungselementes entspricht somit in vorteilhafter Weise der Lage der theoretischen Drehachse. Diese wandert mit der Horizontalverschiebung der Schalenhälften lediglich etwas in vertikaler Richtung. Während der Rotation der Schalenhälften wird die Position der Drehachse nicht verändert. Das mittlere Verbindungselement ist dadurch das einzige annähernd ortsfeste Element an der gesamten Schale und eignet sich hiermit in bevorzugter Weise zur Befestigung anderer Orthesen-Elemente, wie beispielsweise einer Bandage.

Die Langlöcher können gerade oder gekrümmt sein. In einer bevorzugten Ausführungsform sind die waagerecht oder nahezu waagerecht angeordneten Langlöcher gekrümmt, insbesondere leicht gekrümmt. In einer bevorzugten Ausführungsform sind die senkrecht oder nahezu senkrecht angeordneten Langlöcher und die schräg angeordneten Langlöcher nicht gekrümmt.

In einer bevorzugten Ausführungsform sind die Verbindungselemente als Schrauben-Mutter Verbindung ausgebildet. Dem Fachmann sind weitere geeignete Verbindungselemente bekannt, beispielsweise Schnellspannvorrichtungen.

In einer bevorzugten Ausführungsform weist die Rückenschale einen Bauchgurt oder ein Zuggurtsystem auf.

Die vorliegende Erfindung betrifft auch eine Rückenorthese, enthaltend eine erfindungsgemäße orthopädische Rückenschale.

Offenbart ist auch die Verwendung einer erfindungsgemäßen orthopädischen Rückenschale als Rückenorthese.

Die vorliegende Erfindung betrifft auch die Verwendung einer erfindungsgemäßen orthopädischen Rückenschale als Plattform für eine Schulterorthese. Dem Fachmann sind mögliche Aufbauten und Konstruktionen bekannt, mit denen eine Schulterorthese an eine Rückenschale befestigt werden kann.

Die nachfolgend beschriebenen Figuren illustrieren eine Ausführungsform der Erfindung in Form einer variablen Rückenschale mit zwei Seitenschalenteilen. Der Erfindungsgedanke ist nicht auf dieses Ausführungsbeispiel beschränkt.
Figur 1 zeigt eine Ausführungsform einer erfindungsgemäßen Rückenschale in einer mittleren Einstellung;
Figur 2 zeigt die Seitenansicht der erfindungsgemäßen Rückenschale in einer mittleren Einstellung aus Figur 1;
Figur 3 zeigt die erfindungsgemäße Rückenschale aus Figur 1 in der maximalen Einstellung;
Figur 4 zeigt die erfindungsgemäße Rückenschale aus Figur 1 in der maximalen Einstellung bei einem Beckenschiefstand;
Figur 5 zeigt die erfindungsgemäße Rückenschale aus Figur 1 in der minimalen Einstellung bei einem Beckenschiefstand.

### Beispiel 1: Ausführungsform einer erfindungsgemäßen Rückenschale aus zwei Seitenschalenteilen mit jeweils drei Langlöchern

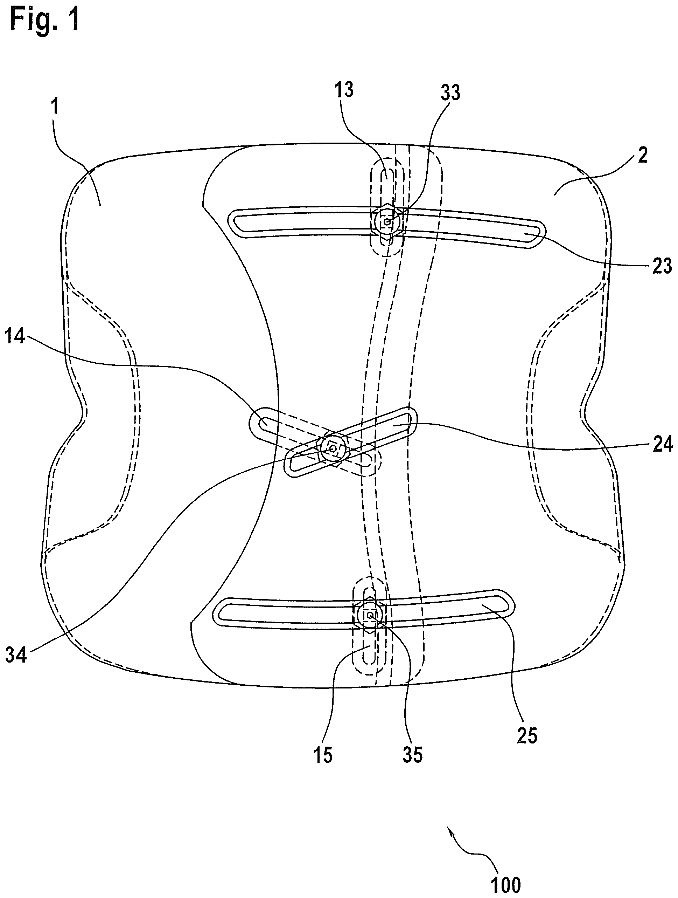

Die Figuren 1 bis 5 zeigen eine erfindungsgemäße orthopädische Rückenschale (100) mit einem ersten Seitenschalenteil (1) und einem zweiten Seitenschalenteil (2), wobei sowohl das erste Seitenschalenteil (1) drei übereinander angeordnete Langlöcher (13, 14, 15) als auch das zweite Seitenschalenteil (2) drei übereinander angeordnete Langlöcher (23, 24, 25) aufweisen und das erste Seitenschalenteil (1) und das zweite Seitenschalenteil (2) über drei Verbindungselemente (31, 32, 33) miteinander verbunden sind, wobei jeweils eins der drei Verbindungselemente (33, 34, 35) durch ein Langloch (13, 14, 15) des ersten Seitenschalenteils (1) und ein Langloch (23, 24, 25) des zweiten Seitenschalenteils (2) hindurchreicht, und wobei das oberste Langloch (13) des ersten Seitenschalenteils (1) und das oberste Langloch (23) des zweiten Seitenschalenteils (2) einen unterschiedlichen Winkel haben und wobei das mittlere Langloch (14) des ersten Seitenschalenteils (1) und das mittlere Langloch (24) des zweiten Seitenschalenteils (2) einen unterschiedlichen Winkel haben und wobei das unterste Langloch (15) des ersten Seitenschalenteils (1) und das unterste Langloch (25) des zweiten Seitenschalenteils (2) einen unterschiedlichen Winkel haben.

Das oberste Langloch (13) und das unterste Langloch (15) des ersten Seitenschalenteils (10) sind senkrecht oder nahezu senkrecht angeordnet und das oberste Langloch (23) und das unterste Langloch (25) des zweiten Seitenschalenteils (2) sind waagerecht oder nahezu waagerecht angeordnet. Das mittlere Langloch (14) des ersten Seitenschalenteils (1) und das mittlere Langloch (24) des zweiten Seitenschalenteils (2) sind schräg angeordnet.

Das oberste Langloch (23) und das unterste Langloch (25) des zweiten Seitenschalenteils (2) sind leicht gekrümmt.

Die zwei Schalenhälften (1,2) können sowohl gegeneinander translatorisch horizontal bewegt als auch gegeneinander rotiert werden. Die Rotation findet dabei um die menschliche Sagittalachse statt, womit lediglich die Achsrichtung von hinter dem Körper nach vor dem Körper gemeint ist. Die Position der Achse ist horizontal mittig und vertikal bedingungsweise beweglich.

Die beiden Schalenhälften (1,2) besitzen demzufolge eine gemeinsame, theoretische Drehachse, die selbst bei horizontaler Bewegung beider Hälften zueinander ihre horizontale Mittenposition beibehält.

Die Verbindung und Führung der zwei Teile (1,2) erfolgt mittels der eingebrachten Langlöcher (13, 14, 15, 23, 24, 25), in denen Verbindungselemente (33, 34, 35) laufen, welche für die relative Bewegung der zwei Teile (1, 2) zueinander (ähnlich einem Kurvengetriebe) nur vordefinierte Bahnen zulassen.

Die Langlöcher (13, 14, 15, 23, 24, 25) sind derart angeordnet, dass sich diejenigen einer Schalenhälfte (1) stets mit denjenigen der anderen Schalenhälfte (2) überkreuzen, um mit dem sich daraus ergebenden Schnittpunkt eine definierte Position des hindurch ragenden Verbindungselementes (33, 34, 35) zu garantieren.

Dabei weist jede Hälfte (1, 2) drei Langlöcher (13, 14, 15, 23, 24, 25) auf, jeweils einen oben, unten und in der Mitte, wobei die reine Führungsfunktion theoretisch auch schon ohne das obere Langloch (13. 23) erfüllt werden könnte. Dies könnte sich allerdings ungünstig auf die gesamte Schalenstabilität auswirken. Die äußeren Langlöcher (13, 23) der linken Hälfte (1) sind vertikal, die äußeren Langlöcher (23, 25) der rechten Hälfte (2) tendenziell horizontal ausgerichtet, denn sie sind nicht exakt waagerecht, sondern leicht bogenförmig ausgeführt.

Der große Radius dieses Bogens ergibt sich aus einem Kompromiss aus dem Abstand zur theoretischen Drehachse und der von der Konstruktion her gewünschten Kurve, welche sich gestaltsmäßig an der Formführung des Schalenrandes orientiert.

Der Ausgleich dieses Kompromisses wird dadurch erreicht, dass die Vertikallanglöcher (13, 15) der linken Schalenhälfte (1) eben nicht punktuell (als Loch) ausgeführt, sondern in der Vertikalrichtung ausgedehnt sind und somit die Horizontallanglöcher (23, 25) bereichsweise überstreifen. Die Grenze der Relativbewegung beider Hälften (1,2) wird demzufolge durch die jeweiligen Enden der Vertikallanglöcher (13, 15) sowie auch der Horizontallanglöcher (23, 25) gebildet.

Die mittleren Langlöcher (14, 24) beider Schalenhälften (1, 2) überlagern sich ebenfalls in einem einzigen Schnittpunkt. In diesem Fall sind beide Langlöcher (14, 24) tendenziell horizontal angeordnet, jedoch etwas diagonal, geradlinig und dabei zueinander spiegelbildlich zur Vertikalachse. So befindet sich dieser mittlere Schnittpunkt und damit auch das zugehörige Verbindungselement (34) stets horizontal in der Mitte.

Die Position des mittleren Verbindungselementes (34) entspricht somit der Lage der theoretischen Drehachse. Diese wandert mit der Horizontalverschiebung der Schalenhälften (1, 2) lediglich etwas in vertikaler Richtung, und zwar in dem Maße, wie groß die vertikale Ausdehnung bzw. der Gabelwinkel der diagonalen mittleren Langlöcher (14, 24) ist. Während der Rotation der Schalenhälften (1, 2) wird die Position der Drehachse nicht verändert.

Das mittlere Verbindungselement (34) ist dadurch das einzige annähernd ortsfeste Element an der gesamten Schale (100) und eignet sich hiermit potentiell am besten zur Befestigung anderer Orthesen-Elemente, wie beispielsweise der Bandage.

Insbesondere durch die Funktion des Zusammenschiebens, besser Ineinanderschiebens der Seitenschalen (1, 2) in horizontaler Richtung sollte dabei aufgrund der runden, rumpfähnlichen Gestalt ein sehr flexibles Schalenmaterial gewählt werden, welches jedoch auch genügend Zähigkeit aufweist, um die ursprünglich beabsichtigte Stützfunktion der Rückenschale (100) zu gewährleisten.

Die Überlappung beider Schalenhälften (1, 2) wird in vorteilhafter Weise durch ein gegenseitiges Ausweichen in der Tiefe erreicht.

Durch als Klemmmechanismus ausgebildete Verbindungselemente (33, 34, 35) lässt sich eine passende Einstellung schließlich arretieren und die Rückenschale (100) zum Gebrauch bereitstellen.

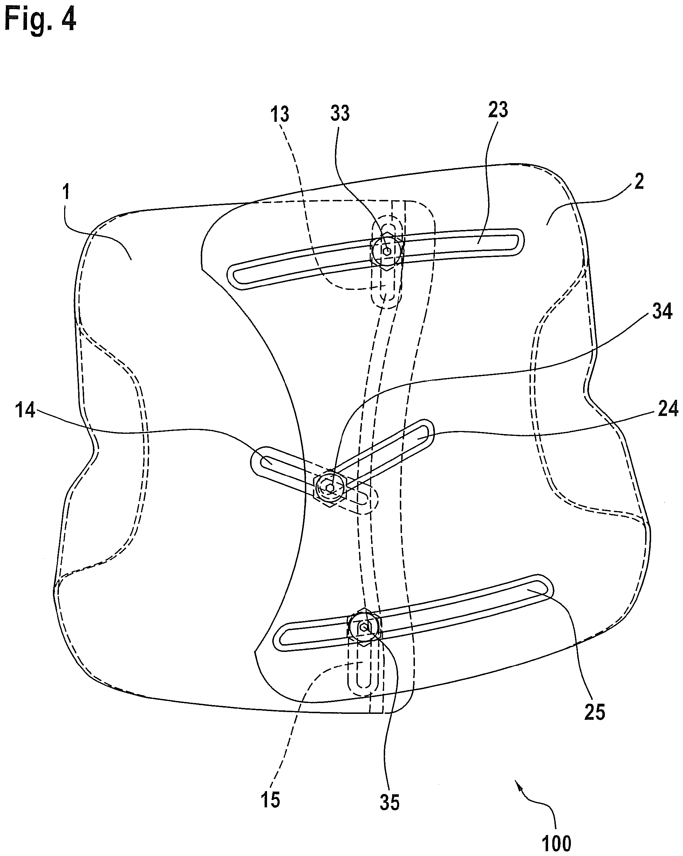

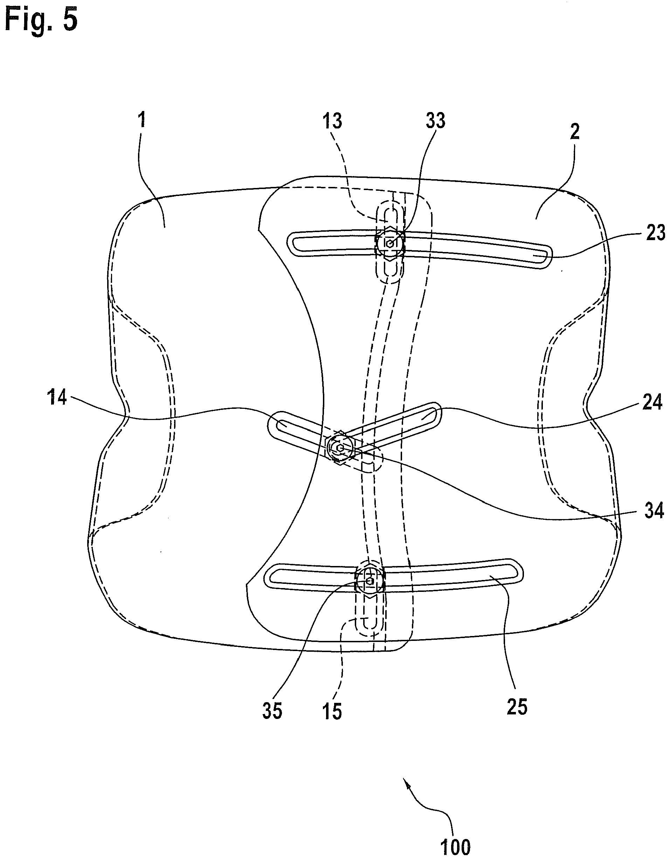

Ein weiterer Vorteil der vorstehenden Anordnung liegt in dem Vermögen der Rückenschale (100), sich an eventuell vorhandene Beckenschiefstände besser anzupassen, sodass beim Tragen der Orthese keine unnötigen Druckstellen entstehen. Das Potential zur Anpassung dahingehend erwächst aus einer Verschiebbarkeit der beiden Schalenhälften (1, 2) in rein vertikaler Richtung gegeneinander. Durch Kombinieren letzterer Funktion mit der gezeigten Rotation erweitert sich das Ausmaß dieser Form der Anpassung nochmals, wie die Figuren 4 und 5 belegen.

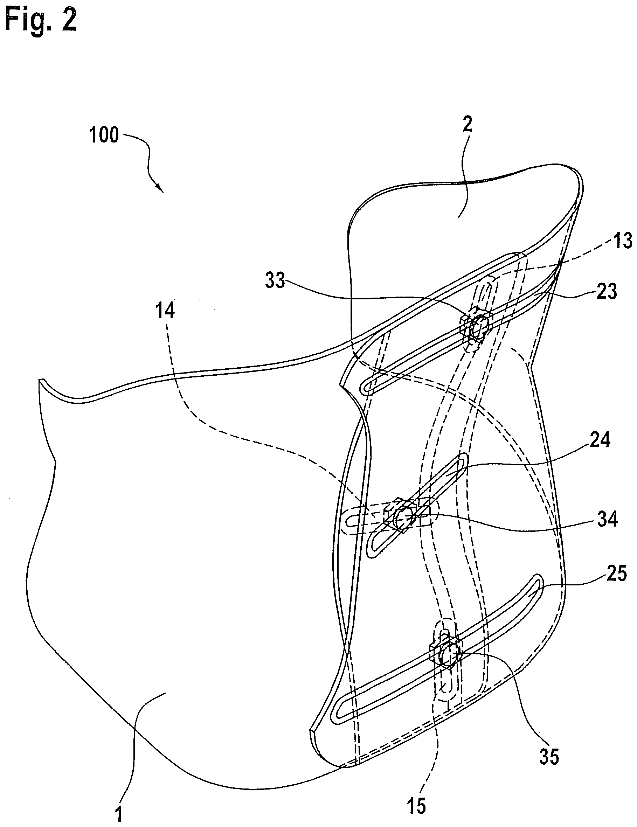

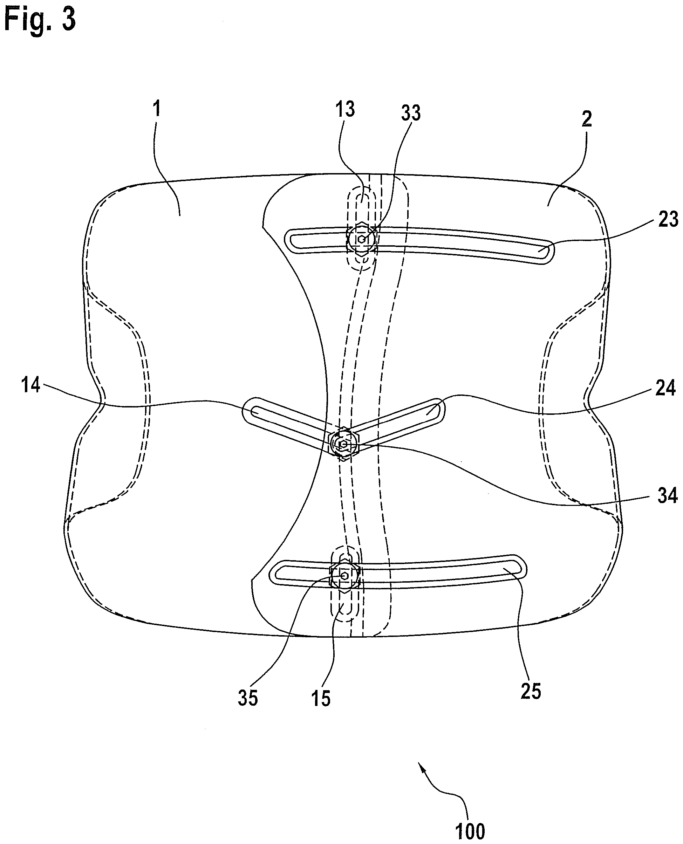

Figur 1 zeigt die Rückenschale (100) in einer mittleren Einstellung. Figur 2 zeigt die Seitenansicht der Rückenschale (100) in der mittleren Einstellung. Figur 3 zeigt die Rückenschale (100) aus Figur 1 in der maximalen Einstellung.

Figur 4 zeigt die Rückenschale (100) in der maximalen Einstellung bei einem Beckenschiefstand und Figur 5 die Rückenschale (100) in der minimalen Einstellung bei einem Beckenschiefstand.

### Beispiel 2: Positionierung und Länge der Langlöcher

Eine vorteilhafte Positionierung und Länge der Langlöcher an den zwei Schalenhälften und eine gezielte Anordnung derselben zum Zweck einer Verstellbarkeit bzw. Adaptierbarkeit der horizontalen Dimension des Konstrukts sowie des Winkels zwischen den beiden Schalenhälften bei einer Rotation um die menschliche Sagittalachse kann ohne Weiteres durch ein mathematische Modell bestimmt werden. Es kann dabei eine Positionierung der Langlöcher gefunden werden, die ein System zur Verbindung und gezielten Führung der beiden Schalenhälften bereitstellt, welches neben der Führungseigenschaft zudem auch bisher unerreichte Flexibilität bietet.

Bestimmend ist dahingehend das Vorhandensein verschieden ausgerichteter Langlöcher in jedem der beiden Schalenteile, welche sich beim Verstellen gegeneinander verschieben oder verdrehen und durch welche hindurch ein speziell konstruiertes Verbindungsteil gesteckt wird, das beide Schalenteile zusammenhält. Dazu sollen sich in vorteilhafter Weise beide Hälften zu jedem Zeitpunkt zumindest so weit überschneiden, dass eine Verbindung derselben durch die Schlitze überhaupt ermöglicht ist. Dadurch, dass zwei korrespondierende Langlöcher in ihrer Längsrichtung immer unterschiedlich ausgerichtet sind, ergibt sich eine fixe Lage des entsprechenden durchgesteckten Verbindungsteils.

Weiterführend existiert ein systematischer Zusammenhang zwischen der horizontalen bzw. vertikalen Position sowie der Länge der Langlöcher. Die exakte Ausführung letzterer ist aber auch in dieser Hinsicht wieder ein Kompromiss zwischen einem mathematischen System und technisch günstigen Gestaltungsparametern.

Die genaue Positionierung und Länge der Langlöcher kann durch einen Fachmann anhand eines geeigneten mathematischen Modells ohne weiteres berechnet werden.

Ein vorteilhaftes Modell geht von geradlinigen anstelle gebogener Horizontallanglöcher aus, um der Kernproblematik nahezukommen. Der Einfluss der Horizontalvergrößerung ist im gesamten System dominanter als die Rotation, da letztere sich lediglich im Winkelbereich von -30° bis +30° abspielt. Die Horizontalverstellung dagegen findet je nach Schalentyp im Bereich -40/80mm bis +40/80mm statt. Die Ausführung der Horizontallanglöcher als leichter Bogen in der Praxis kommt allerdings der Rotationsbewegung etwas entgegen. Die verbleibenden sich noch ergebenden Positionsdifferenzen werden durch die Längsausdehnung der Vertikallanglöcher abgefangen.

## Patentansprüche

1. Orthopädische Rückenschale (100), wobei die Rückenschale (100) mindestens ein erstes flexibles Seitenschalenteil (1) und mindestens ein zweites flexibles Seitenschalenteil (2) aufweist, wobei das erste Seitenschalenteil (1) und das zweite Seitenschalenteil (2) über mindestens zwei Verbindungselemente miteinander verbunden sind und wobei das erste Seitenschalenteil (1) und das zweite Seitenschalenteil (2) über die mindestens zwei Verbindungselemente variabel aneinander fixiert werden können, wobei sowohl das erste Seitenschalenteil (1) mindestens zwei übereinander angeordnete Langlöcher (13, 15) als auch das zweite Seitenschalenteil (2) mindestens zwei übereinander angeordnete Langlöcher (23, 25) aufweisen und das erste Seitenschalenteil (1) und das zweite Seitenschalenteil (2) über die mindestens zwei Verbindungselemente (33, 35) miteinander verbunden sind, wobei jeweils eins der zwei Verbindungselemente (33, 35) durch ein Langloch (13, 15) des ersten Seitenschalenteils (1) und ein Langloch (23, 25) des zweiten Seitenschalenteils (2) hindurchreicht, und wobei das oberste Langloch (13) des ersten Seitenschalenteils (1) und das oberste Langloch (23) des zweiten Seitenschalenteils (2) einen unterschiedlichen Winkel zueinander haben und wobei das unterste
Langloch (15) des ersten Seitenschalenteils (1) und das unterste Langloch (25) des zweiten Seitenschalenteils (2) einen unterschiedlichen Winkel zueinander haben, **dadurch gekennzeichnet, dass** das
oberste Langloch (13) und das unterste Langloch (15) des ersten Seitenschalenteils (1) senkrecht oder nahezu senkrecht angeordnet sind, wobei das oberste Langloch (23) und das unterste Langloch (25) des zweiten Seitenschalenteils (2) waagerecht oder nahezu waagerecht angeordnet sind.

2. Orthopädische Rückenschale nach Anspruch 1, wobei sowohl das erste Seitenschalenteil (1) mindestens drei übereinander angeordnete Langlöcher (13, 14, 15) als auch das zweite Seitenschalenteil (2) mindestens drei übereinander angeordnete Langlöcher (23, 24, 25) aufweisen.

3. Orthopädische Rückenschale nach Anspruch 2, wobei das mittlere Langloch (14) des ersten Seitenschalenteils (1) und das mittlere Langloch (24) des zweiten Seitenschalenteils (2) einen unterschiedlichen Winkel haben.

4. Orthopädische Rückenschale nach einem der vorhergehenden Ansprüche, wobei das erste Seitenschalenteil (1) und das zweite Seitenschalenteil (2) über überlappende Flächenbereiche (11, 12) miteinander verbunden sind.

5. Orthopädische Rückenschale nach einem der vorhergehenden Ansprüche, wobei das erste Seitenschalenteil (1) und das zweite Seitenschalenteil (2) jeweils mindestens einen Teil des Rückens und eine Taillenseite umfassen können.

6. Orthopädische Rückenschale nach einem der vorhergehenden Ansprüche, wobei das erste Seitenschalenteil (1) und das zweite Seitenschalenteil (2) aus einem flexiblen Kunststoffmaterial ausgebildet sind.

7. Orthopädische Rückenschale nach einem der vorhergehenden Ansprüche, wobei das mindestens eine Verbindungselement als Schrauben-Mutter Verbindung, als Schnellspannvorrichtung oder als Nietenverbindung ausgebildet ist.

8. Orthopädische Rückenschale nach einem der vorhergehenden Ansprüche, wobei das mindestens eine Verbindungselement als Clipverbindung, als Klettverbindung oder als schiebbare Rastverbindung ausgebildet ist.

9. Orthopädische Rückenschale nach einem der vorhergehenden Ansprüche, wobei das mittlere Langloch (14) des ersten Seitenschalenteils (1) und das mittlere Langloch (24) des zweiten Seitenschalenteils (2) schräg angeordnet sind.

10. Orthopädische Rückenschale nach einem der vorhergehenden Ansprüche, wobei die Rückenschale einen Bauchgurt oder ein Zuggurtsystem aufweist.

11. Rückenorthese, enthaltend eine orthopädische Rückenschale nach einem der Ansprüche 1 bis 10.

12. Verwendung einer orthopädischen Rückenschale nach einem der Ansprüche 1 bis 10 als Plattform zur Befestigung an eine Schulterorthese.

## Claims

1. An orthopedic back shell (100), said back shell (100) comprising at least one first flexible side shell part (1) and at least one second flexible side shell part (2), wherein the first side shell part (1) and the second side shell part (2) are connected to one another via at least two connector elements and wherein the first side shell part (1) and the second side shell part (2) can be fixed to one another in a variable fashion via the at least two connector elements, wherein the first side shell part (1) comprises at least two oblong holes (13, 15) disposed one above the other and wherein the second side shell part (2) comprises at least two oblong holes (23, 25) disposed one above the other, and wherein the first side shell part (1) and the second side shell part (2) are connected to one another via the at least two connector elements (33, 35), and wherein each of the two connector elements (33, 35) extend through one oblong hole (13, 15) of the first side shell part (1) and one oblong hole (23, 25) of the second side shell part (2), and wherein the uppermost oblong hole (13) of the first side shell part (1) and the uppermost oblong hole (23) of the second side shell part (2) have a different angle, and wherein the lowermost oblong hole (15) of the first side shell part (1) and the lowermost oblong hole (25) of the second side shell part (2) have a different angle, **characterized in that** the uppermost oblong hole (13) and the lowermost oblong hole (15) of the first side shell part (1) are disposed vertically or virtually vertically, wherein the uppermost oblong hole (23) and lowermost oblong hole (25) of the second side shell part (2) are disposed horizontally or virtually horizontally.

2. The orthopedic back shell according to Claim 1, wherein the first side shell part (1) comprises at least three oblong holes (13, 14, 15) disposed one above the other and the second side shell part (2) comprises at least three oblong holes (23, 24, 25) disposed one above the other.

3. The orthopedic back shell according to Claim 2, wherein the middle oblong hole (14) of the first side shell part (1) and the middle oblong hole (24) of the second side shell part (2) have a different angle.

4. The orthopedic back shell according to one of the previous claims, wherein the first side shell part (1) and the second side shell part (2) are connected to one another via overlapping surface regions (11, 12).

5. The orthopedic back shell according to one of the previous claims, wherein the first side shell part (1) and the second side shell part (2) are each able to surround at least part of the back and a side of the torso.

6. The orthopedic back shell according to one of the previous claims, wherein the first side shell part (1) and the second side shell part (2) are made of a flexible plastic material.

7. The orthopedic back shell according to one of the previous claims, wherein the at least one connector element is embodied as a nut-and-bolt connection, as a quick-clamping mechanism, or as a rivet connection.

8. The orthopedic back shell according to one of the previous claims, wherein the at least one connector element is embodied as a clip connection, as a Velcro connection, or as a slidable latching connection.

9. The orthopedic back shell according to one of the previous claims, wherein the middle oblong hole (14) of the first side shell part (1) and the middle oblong hole (24) of the second side shell part (2) are disposed obliquely.

10. The orthopedic back shell according to one of the previous claims, wherein the back shell has an abdominal belt or a tension band system.

11. A back orthotic comprising an orthopedic back shell according to one of claims 1 to 10.

12. Use of an orthopedic back shell according to one of claims 1 to 10 as a platform for the fixation to a shoulder orthotic.

## Revendications

1. Coque dorsale (100) orthopédique, la coque dorsale (100) comportant au moins une première partie de coque latérale (1) flexible et au moins une seconde partie de coque latérale (2) flexible, la première partie de coque latérale (1) et la seconde partie de coque latérale (2) étant reliées l'une à l'autre par au moins deux éléments de liaison et la première partie de coque latérale (1) et la seconde partie de coque latérale (2) pouvant être fixées l'une à l'autre de manière variable par le biais des au moins deux éléments de liaison, la première partie de coque latérale (1) comportant au moins deux trous oblongs (13, 15) disposés l'un au-dessus de l'autre et la seconde partie de coque latérale (2) comportant aussi au moins deux trous oblongs (23, 25) disposés l'un au-dessus de l'autre et la première partie de coque latérale (1) et la seconde partie de coque latérale (2) étant reliées l'une à l'autre par les au moins deux éléments de liaison (33, 35), respectivement un des deux éléments de liaison (33, 35) traversant un trou oblong (13, 15) de la première partie de coque latérale (1) et un trou oblong (23, 25) de la seconde partie de coque latérale (2), et le trou oblong supérieur (13) de la première partie de coque latérale (1) et le trou oblong supérieur (23) de la seconde partie de coque latérale (2) ayant un angle différent l'un par rapport à l'autre et le trou oblong inférieur (15) de la première partie de coque latérale (1) et le trou oblong inférieur (25) de la seconde partie de coque latérale (2) ayant un angle différent l'un par rapport à l'autre, **caractérisé en ce que** le trou oblong supérieur (13) et le trou oblong inférieur (15) de la première partie de coque latérale (1) sont disposés verticalement ou presque verticalement, le trou oblong supérieur (23) et le trou oblong inférieur (25) de la seconde partie de coque latérale (2) étant disposés horizontalement ou presque horizontalement.

2. Coque dorsale orthopédique selon la revendication 1, dans laquelle la première partie de coque latérale (1) comporte au moins trois trous oblongs (13, 14, 15) disposés les uns au-dessus des autres et la seconde partie de coque latérale (2) comporte aussi au moins trois trous oblongs (23, 24, 25) disposés les uns au-dessus des autres.

3. Coque dorsale orthopédique selon la revendication 2, dans laquelle le trou oblong central (14) de la première partie de coque latérale (1) et le trou oblong central (24) de la seconde partie de coque latérale (2) ont un angle différent.

4. Coque dorsale orthopédique selon l'une des revendications précédentes, dans laquelle la première partie de coque latérale (1) et la seconde partie de coque latérale (2) sont reliées l'une à l'autre par le biais de zones de surface (11, 12) se chevauchant.

5. Coque dorsale orthopédique selon l'une des revendications précédentes, dans laquelle la première partie de coque latérale (1) et la seconde partie de coque latérale (2) peuvent comprendre respectivement au moins une partie du dos et un côté de la taille.

6. Coque dorsale orthopédique selon l'une des revendications précédentes, dans laquelle la première partie de coque latérale (1) et la seconde partie de coque latérale (2) sont réalisées à partir d'une matière plastique flexible.

7. Coque dorsale orthopédique selon l'une des revendications précédentes, dans laquelle l'au moins un élément de liaison est réalisé sous la forme d'un assemblage vis-écrou, d'un dispositif de serrage rapide ou d'un assemblage par rivet.

8. Coque dorsale orthopédique selon l'une des revendications précédentes, dans laquelle l'au moins un élément de liaison est réalisé sous la forme d'un assemblage par pince, d'un assemblage par ruban autoagrippant ou d'un assemblage coulissant à encliquetage.

9. Coque dorsale orthopédique selon l'une des revendications précédentes, dans laquelle le trou oblong central (14) de la première partie de coque latérale (1) et le trou oblong central (24) de la seconde partie de coque latérale (2) sont disposés en oblique.

10. Coque dorsale orthopédique selon l'une des revendications précédentes, dans laquelle la coque dorsale comporte une sangle ventrale ou un système de sangle à tirer.

11. Orthèse dorsale, comprenant une coque dorsale orthopédique selon l'une des revendications 1 à 10.

12. Utilisation d'une coque dorsale orthopédique selon l'une des revendications 1 à 10 en tant que plate-forme pour la fixation à une orthèse d'épaule.
